# EUROPÄISCHE PATENTSCHRIFT

(11) **EP 0 527 241 B1**
(45) Veröffentlichungstag und Bekanntmachung des Hinweises auf die Patenterteilung: **16.03.1994**
(21) Anmeldenummer: 91111738.0
(22) Anmeldetag: 15.07.1991
(51) Int. Cl.: A61K 31/375, A61K 9/06, A61K 9/20

(54) **Verwendung der Ascorbinsäure zur Zubereitung von Arzneimitteln zur Anwendung im Genitalbereich**
The use of Ascorbic acid for the preparation of medicaments for use in the genital region
L'utilisation d'acide ascorbique pour la préparation d'un médicament pour utilisation dans la région génitale

(43) Veröffentlichungstag der Anmeldung: 17.02.1993
(73) Patentinhaber: Artesan Pharma Gesellschaft mit beschränkter Haftung, D-29439 Lüchow (DE)
(72) Erfinder: Hotzel, Knut A., W-3130 Lüchow (DE); Petersen, Eiko, Prof. Dr. med., W-7800 Freiburg (DE)
(74) Vertreter: von Raffay, Vincenz, Dipl.-Ing.

(56) Entgegenhaltungen:
- WO-A-87/04069
- WO-A-88/04176
- FR-A- 2 646 604
- US-A- 4 711 780
- PATENT ABSTRACTS OF JAPAN, Band 10, Nr. 230 (C-365)[2286], 9. August 1986,Seite 111 C 365; & JP-A-61 65 822 (KAO CORP.) 04-04-1986
- ROTE LISTE, 1987, Editio Cantor, Aulendorf, DE;
- AMERICAN JOURNAL OF OBSTETRICS AND GYNECOLOGY, Band 151, Nr. 7, 1. April 1985,Seiten 976-980, The C.V. Mosby Company, St. Louis, Missouri, US; S.L. ROMNEY etal.: "Plasma vitamin C and unterine cervical dysplasia"
- GINEKOLOGIA POLSKA, Band 40, Nr. 7, 1969, Seiten 773-778, PL; B. HOFFMANN etal.: "The vitamin C concentrations in the cervical mucus in women infested anduninfested by trichomonas vaginalis"
- Rote Liste, 1987, No. 45126
- Rote Liste, 1949, Seite 590, "Spuman"
- Rote Liste, 1952, Seite 653, "Tampovagan"

## Beschreibung

Die Erfindung betrifft die Verwendung von Ascorbinsäure, sowie Ascorbinsäure enthaltende Arzneizubereitungen.

Ascorbinsäure ist im Stand der Technik bekannt (vgl. Römpp, Chemielexikon, 9. erweiterte und neu bearbeitete Auflage, Bd. 1, 1989, S. 265 ff.). Im Bereich der Medizin ist die typische C-Avitaminose der Skorbut, von dem auch der Name Ascorbinsäure hergeleitet ist. Therapeutisch wird Vitamin C eingesetzt zur Prophylaxe und Bekämpfung von Skorbut. Schäden, nach Operationen, zur Unterstützung der Resorption von oral verabreichtem Eisen, zur schnelleren Heilung von Knochenbrüchen, als allgemeines Roborans. Höhere Dosen an Vitamin C sind viel als Vorbeugungsmittel gegen Erkältung, zur Beschleunigung der Wundheilung, zur günstigen Beeinflussung von Rheuma, Polyarthritis und Tuberkulose sowie der Konzentrations- und Reaktionsfähigkeit empfohlen worden (siehe Römpp, S. 4541).

In technischer Hinsicht wird Ascorbinsäure als Antoxidanz für technische, vor allem aber für lebensmitteltechnische Zwecke verwendet. Die Nützlichkeit hoher Ascorbinsäuredosen gegen Schnupfen und gegen Krebs wird immer noch angezweifelt. Allerdings sollen positive Auswirkungen auf Wundheilung und Immunsystem vorhanden sein.

Obwohl die Ascorbinsäure/Vitamin C jahrzehntelang bekannt ist, finden sich bis zum heutigen Tage nur unzureichende Hinweise auf seine Wirkung im gynäkologischen Bereich (vgl. WO-A-87/04069 und WO-A-88/04176). Im *American Journal* *of Obstetriccs and Gynecology*, 151 (7), 1. April 1985, Seiten 976 bis 980, The C.V. Mosby Company, St. Louis, Missouri, US., wird aufgezeigt, daß asymptomatische Patientinnen, bei denen cytologisch eine Dysplasie von Zervixepithelzellen nachgewiesen ist, einen erniedrigten Vitamin-C-Gehalt im Plasma aufweisen. Ebenfalls wird dort erwähnt, daß noch weitere Untersuchungen notwendig sind, da der Zusammenhang zwischen Plasmakonzentration und verschiedenen Epitheldysplasien alles andere als geklärt ist. Es werden dann noch weitere Studien genannt, die auch einen Zusammenhang zwischen dem Vitamin-C-Spiegel im Plasma und Dysplasien unterstützen und die sozusagen angeführt werden, um das Thema Vitamin-C- und Dysplasie zu aktualisieren und mehr Informationen zu erhalten, wobei dieser Bericht mit noch offenen Fragen abschließt und weitere Untersuchungen fordert. Mit keinem Wort wird in diesem Dokument die lokale Zufuhr von Vitamin C in die Vagina und an den Muttermund angesprochen oder irgendwie nahegelegt. Da bei den Lebewesen, Zusafz insbesondere beim Menschen, im Genitalbereich die unterschiedlichsten Beschwerden und Erkrankungen auftreten, besteht ein erheblicher Bedarf an einem einfachen Arzneimittel bzw. einer Arzneizubereitung, die hier Abhilfe schaffen kann. Überraschenderweise gelingt dies erfindungsgemäß durch die Verwendung von Ascorbinsäure gemäß Anspruch 1.

Es hat sich gezeigt, daß folgende Erkrankungen im Genitalbereich wirsksam behandelt werden können:

### 1. Dysplasien im Bereich des Muttermundes, wenn sie Folge einer Papillomavirus-Infektion sind:

Es ist bekannt, daß das Rauchen einer der Risikofaktoren für die Entstehung eines Genitalkrebses ist. Möglicherweise hängt dies mit dem relativen Vitamin C-Defizit der Raucher zusammen, denn sie haben einen um 40% höheren Vitamin C-Bedarf als Nichtraucher.
Untersuchungen an einer Patientin mit einer schweren Dysplasie der Zervix (Pap. IV) haben unter der erfindungsgemäßen Vitamin C-Behandlung zu einer Normalisierung des zytologischen Abstriches (Pap. II) geführt.
Gleichzeitig sind die durch die Papillomaviren hervorgerufenen Kondylome und ihre Entzündungsreaktion auf der Zervix verschwunden.

### 2. Störung der Vaginalflora (z.B. Aminkolpitis), bei der die normale Laktobazillenflora durch Gardnerella vaginalis, Anaerobier und andere Bakterien verdrängt wurde.

Die bakteriell gestörte Vaginalflora mit unangenehm riechendem Ausfluß ist häufig, etwa 10% der Frauen haben eine derartige Störung und leiden unter diesem scheinbar ästhetischen Problem. Durch die hohe Keimzahl fakultativ pathogener Bakterien besteht aber zusätzlich ein erhöhtes Entzündungsrisiko in der Schwangerschaft und nach operativen Eingriffen oder im Gefolge von sexuell übertragenen Erregern, die die Fähigkeit besitzen, in den inneren Genitalbereich zu gelangen und dort Infektionen auszulösen.

Die bisherigen Untersuchungen haben gezeigt, daß diese bakterielle Störung durch viele verschiedene Medikamente, die sowohl per os als auch lokal angewendet werden, nicht immer beseitigt werden kann. Hinzu kommt, daß infolge einer Schwäche der individuellen Normalflora diese Störung bei vielen dieser Frauen sehr rasch wieder auftritt. Es wird daher nach Behandlungsmöglichkeiten gesucht, die harmlos sind und wiederholt angewendet werden können und die von der Patientin auch toleriert werden.
Es ist bekannt, daß durch die Ansäuerung des Scheidenbereiches die Normalflora bereits soweit begünstigt wird, daß es hierdurch in vielen Fällen zu einer Normalisierung der Vaginalflora kommt.
Untersuchungen mit der erfindungsgemäßen Gabe von Vitamin C in Tablettenform in die Scheide bei diesen bakteriellen Störungen haben gezeigt, daß hierdurch eine Normalisierung der Vaginalflora zu erreichen ist.
Die Akzeptanz dieser Behandlung durch die Patientin ist sehr hoch.
Untersuchungen in vitro unterstützen die klinische Beobachtung, daß die Normalflora, d.h. die Laktobazillen, unter Vitamin C weniger gehemmt werden als andere Bakterien, z.B. Darmkeime.

### 3. Eitrige Kolpitis (Entzündung der Scheide), bei der bis heute kein typischer Erreger nachweisbar ist, die vermutlich durch eine Virusinfektion verursacht ist:

Die eitrige Kolpitis ist ein bis heute ungelöstes Problem, welches die junge Patientin, die es meistens betrifft, sehr belastet. Spezielle Erreger für diese Störung sind bis heute nicht bekannt. Möglicherweise handelt es sich um eine Virusinfektion. Meist liegt gleichzeitig eine Störung der Vaginalflora vor. Die lokale erfindungsgemäße Anwendung von Vitamin C wirkt sowohl gegen die Virusinfektion (Aktivierung der Immunabwehr) als auch auf die Beseitigung der fakultativ pathogenen Bakterien durch die Ansäuerung.

Erfindungsgemäße Langzeitanwendungen von Vitamin C bei Frauen mit dieser Störung haben es zu einer deutlichen Verbesserung der Beschwerden bis hin zur Heilung gebracht. Dabei ist die Akzeptanz der Therapie durch die Patientin, die häufig seit Jahren an dieser Erkrankung leiden, auch über viele Wochen hervorragend.

Für die verschiedenen Anwendungsgebiete sind unterschiedliche Dosierungen notwendig, da einmal eine Aktivierung der humoralen und zellulären Abwehr beabsichtigt ist bei der Beseitigung von Virusinfektionen und zum anderen bei bakteriellen Keimstörungen die Ansäuerung und Hemmung der Bakterien nicht so stark sein darf, daß die Normalflora nicht auswachsen kann. Minimal sollten jedoch ca. 3 Gew% Ascorbinsäure nicht unterschritten und etwa 50 Gew% nicht überschritten werden.
Vorteilhafterweise sind 3 verschiedene Vitamin C-Konzentrationen zwischen 50 und 500 mg pro Tablette oder g der Salbe vorgesehen.

Die Anwendungszeit ist bei den verschiedenen Indikationen unterschiedlich.
Insgesamt wird bei der eitrigen Kolpitis und bei der Papillomavirus-Infektion eine mehrwöchige Therapie erforderlich sein.

Der Vorteil der lokalen erfindungsgemäßen Verwendung von Vitamin C bei der Patientin mit diesen Störungen ist folgender:
- Durch die lokale Applikation werden dort hohe Vitamin C-Konzentrationen erreicht, wo sie benötigt werden.
- Das Vitamin C-Defizit der dysplastischen Zellen ist möglicherweise zu beseitigen und somit wäre ein protektiver Effekt vor weiterer Krebsentwicklung zu erzielen.
- Die lokale Anwendung kann von der Patientin vorgenommen werden, so daß längere Anwendungen leichter möglich sind.
- Die langfristige Ansäuerung des Vaginalbereiches begünstigt die Selektion und Ansiedlung von wichtigen Laktobazillenarten (Normalflora).
- Vitamin C ist völlig atoxisch und kann in hohen Konzentrationen gegeben werden. Überdosierungen sind nicht bekannt.
- Die Akzeptanz auch einer längerfristigen lokalen Anwendung von Vitamin C durch die Patientin ist sehr hoch.
- Eine systemische Aufnahme von Vitamin C aus dem Vaginalbereich ist durchaus erwünscht und führt zusätzlich zu einer erhöhten Vitamin C-Zufuhr.
- Die Gabe von Vitamin C während der Schwangerschaft ist unbedenklich.

Im folgenden werden zwei Ausführungsbeispiele der vorliegenden Erfindung beschrieben:

### Beispiel 1

Eine 12,5 Gew% Vitamin C enthaltende Vaginalsalbe wird wie folgt bei einer Ansatzgröße von 200 kg hergestellt:
- weiße Vaseline: 43 750 g
- Cetylstearylalkohol (Lanette N): 52 500 g
- dünnflüssiges Paraffin: 78 750 g

werden in der Salbenmaschine bei 80° C geschmolzen. Die Schmelze wird 20 Minuten gerührt und homogenisiert. Die Kühlung wird nun eingeschaltet. Bei einer Innentemperatur von 35° C wird der Dissolver ausgeschaltet. Bei einer Innentemperatur von 30° C erfolgt die Zugabe von
- Ascorbinsäure: 25 000 g.

Dissolver 15 Minuten laufen lassen.

Die Mischung wird im Teilvakuum kaltgerührt und anschließend über einen Homogenisator in die Vorratsbehälter für die Abfüllung gegeben.

### Beispiel 2

Eine 5 Gew% Vitamin C enthaltende Vaginaltablette wird wie folgt bei einer Ansatzgröße von 100 000 Tabletten (100 kg) hergestellt: 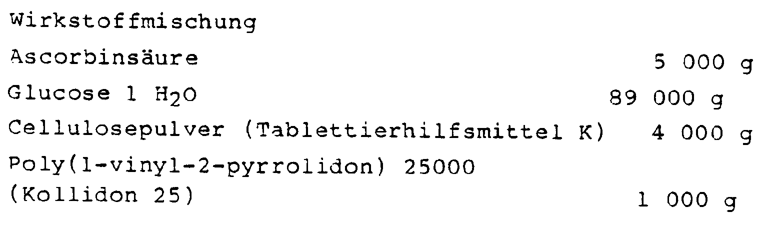
werden fein gesiebt (Frewitt-Siebmaschine),
(1,0 mm Maschenweite) und im Taumelfaß-Mischer in einem V2A-Edelstahlfaß (200 l) 10 Minuten gemischt. Danach wird
- Magnesiumstearat: 1 000 g

von Hand zugesiebt und 2 Minuten im Taumelfaß-Mischer nachgemischt.

## Patentansprüche

1. Verwendung von Ascorbinsäure zur Herstellung von Arzneizubereitungen, insbesondere als Tablette oder Salbe, für die Applikation bei der Aminkolpitis oder eitrigen Kolpitis und der Dysplasie im Bereich des Muttermundes als Folge einer Papillomavirus-Infektion im Genitalbereich von weiblichen Lebewesen.

2. Ascorbinsäure enthaltende Arzneizubereitungen nach Anspruch 1, gekennzeichnet durch eine 3 bis 50 Gew% Ascorbinsäure enthaltende Vaginalsalbe auf Basis von Paraffin, Fettalkohol mit 12 - 24 C-Atomen und Vaseline oder andere Trägerstoffe, die sich zur Applikation im Vaginalbereich eignen.

3. Ascorbinsäure enthaltende Arzneizubereitung nach Anspruch 1, gekennzeichnet durch :
12,5 Gew% Ascorbinsäure
39,4 Gew% dünnflüssiges Paraffin
26,3 Gew% Cetylstearylakohol
21,8 Gew% weiße Vaseline.

4. Ascorbinsäure enthaltende Arzneizubereitung nach Anspruch 1, gekennzeichnet durch eine 5 Gew% Ascorbinsäure enthaltende Vaginaltablette, umfassend
89 Gew% Glucose 1 H₂O
4 Gew% Cellulosepulver als Tablettierhilfsmittel
1 Gew% Polyvinylpyrrolidon
1 Gew% Magnesiumstearat.

## Claims

1. Use of ascorbic acid for the preparation of medicaments, particularly as a tablet or ointment, for use in amine vaginitis or purulent vaginitis and dysplasia in the vicinity of the mouth of the uterus as a result of papilloma virus infection in the genital region in female animals.

2. Ascorbic acid-containing medicaments according to claim 1, characterized by a 3 to 50% by weight ascorbic acid-containing vaginal ointment based on paraffin, fatty alcohol with 12 to 24 C-atoms and vaseline or other carriers, which are suitable for application in the vaginal region.

3. Ascorbic acid-containing medicament according to claim 1, characterized by
12.5% by weight ascorbic acid
39.4% by weight highly liquid paraffin
26.3% by weight cetyl stearyl alcohol
21.8% by weight white vaseline.

4. Ascorbic acid-containing medicament according to claim 1, characterized by a 5% by weight ascorbic acid-containing vaginal tablet comprising
89% by weight glucose 1 H₂O
4% by weight cellulose powder as the tabletting aid
1% by weight polyvinyl pyrrolidone
1% by weight magnesium stearate.

## Revendications

1. Utilisation de l'acide ascorbique pour la fabrication de compositions médicamenteuses, en particulier sous forme de comprimés ou de pommade, pour l'administration dans le cas de l'aminocervicite ou de la cervicite purulente et de la dysplasie dans la région de l'orifice de l'utérus consécutives à une infection par le virus du papillome dans la région génitale d'êtres vivants femelles.

2. Préparations médicamenteuses contenant de l'acide ascorbique selon la revendication 1, caractérisées par une pommade vaginale contenant 3 à 50 % en poids d'acide ascorbique à base de paraffine, d'un alcool gras en C₁₂ à C₂₄ et de vaseline ou d'autres supports convenant pour l'administration dans la région vaginale.

3. Préparation médicamenteuse contenant de l'acide ascorbique selon la revendication 1, caractérisée par :
12,5 % en poids d'acide ascorbique
39,4 % en poids de paraffine fluide
26,3 % en poids d'alcool cétylstéarylique
21,8 % en poids de vaseline blanche.

4. Préparation médicamenteuse contenant de l'acide ascorbique selon la revendication 1, caractérisée par un comprimé gynécologique contenant 5 % en poids d'acide ascorbique, comprenant :
89 % en poids de glucose monohydrate
4 % en poids de poudre de cellulose comme adjuvant de transformation en comprimés
1 % en poids de polyvinylpyrrolidone
1 % en poids de stéarate de magnésium.
